# EUROPÄISCHE PATENTSCHRIFT

(11) **EP 3 440 043 B1**
(45) Veröffentlichungstag und Bekanntmachung des Hinweises auf die Patenterteilung: **13.05.2020**
(21) Anmeldenummer: 16727475.2
(22) Anmeldetag: 02.06.2016
(51) Int. Cl.: C07C 45/51, C07C 45/53, C07C 45/54, C07C 47/21, A23L 27/20, A23L 9/10, A23L 19/18, A23L 27/26, A23L 23/00, A23L 23/10

(54) **VERFAHREN ZUR HERSTELLUNG UNGESÄTTIGTER DECANALE**
METHOD FOR PRODUCING UNSATURATED DECANALS
PROCÉDÉ DE PRÉPARATION DE COMPOSÉS DÉCANAL INSATURÉS

(43) Veröffentlichungstag der Anmeldung: 13.02.2019
(73) Patentinhaber: Symrise AG, 37603 Holzminden (DE)
(72) Erfinder: ONGOUTA, Jekaterina, 37627 Stadtoldendorf (DE); BACKES, Michael, 37603 Holzminden (DE); LEY, Jakob, 37603 Holzminden (DE); KOPPE, Volkmar, 37671 Höxter-Stahle (DE); KOCH, Jens, 37632 Eschershausen (DE)
(74) Vertreter: Eisenführ Speiser
(86) Internationale Anmeldenummer: PCT/EP2016/062571
(87) Internationale Veröffentlichungsnummer: WO 2017/207060

(56) Entgegenhaltungen:
- US-A- 3 686 003
- BLANK IMRE ET AL: "Identification of potent odorants formed by autoxidation of arachidonic acid: Structure elucidation and synthesis of (E,Z,Z)-2,4,7,-tridecatrienal", JOURNAL OF AGRICULTURAL AND FOOD CHEMISTRY, AMERICAN CHEMICAL SOCIETY, US, Bd. 49, Nr. 6, 1. Juni 2001 (2001-06-01), Seiten 2959-2965, XP002502551, ISSN: 0021-8561, DOI: 10.1021/JF010160+ [gefunden am 2001-05-22] in der Anmeldung erwähnt

## Beschreibung

Die vorliegende Erfindung bezieht sich auf Aromazusammensetzungen und Verfahren zur Herstellung derselben.

Die gezielte Herstellung und Verwendung von Aromamischungen bestimmter Geschmacksrichtungen, die lebensmittelrechtlich kompatibel sind, ist Gegenstand ständiger Forschungsanstrengungen.

Im amerikanischen Patent US 3686003 A sind zum Beispiel ein Verfahren zur Herstellung sowie die Verwendung von aliphatischen Aldehyden mit 11 bis 17 Kohlenstoffatomen, um in Lebensmitteln einen würzigen Geschmack zu vermitteln, beschrieben.

Zu den interessanten Geschmacksstoffen zählen dabei ungesättigte Decanale, insbesondere 2E,4Z,7Z und 2E,4E,7Z Tridecatrienal sowie 2E,4E-Decadienal. Diese zeichnen sich in vielen Anwendungen durch einen Fleischgeschmack, insbesondere Hähnchengeschmack aus.

Die Herstellung dieser Aromastoffe erfolgt meist vollsynthetisch aus kleineren Bauteilen mittels Kupplungsreaktionen, wie z.B. in Blank et al. J. Agric. Food Chem. 2001, 2959.

Es stellt sich somit die Aufgabe, alternative und einfachere Herstellungsmethoden zur Darstellung ungesättigter Decanale zu finden.

Diese Aufgabe wird durch das Verfahren gemäß Anspruch 1 gelöst. Demgemäß wird ein Verfahren zur Herstellung ungesättigter Decanale, insbesondere 2E,4Z,7Z und 2E,4E,7Z Tridecatrienal und/oder 2E,4E-Decadienal vorgeschlagen, umfassend die Schritte
a) Luftoxidation eines Arachidonsäure oder Arachidonsäureester enthaltenden Edukts
b) Zersetzung des entstehenden Peroxids
c) Aufkonzentrierung.

Überraschenderweise hat sich herausgestellt, dass so bei vielen Anwendungen der vorliegenden Erfindung insbesondere 2E,4Z,7Z und 2E,4E,7Z Tridecatrienal in einer sensorisch akzeptablen in vielen Fällen sogar präferierten Qualität hergestellt werden kann.

Bei den meisten Anwendungen der vorliegenden Erfindung kann einer oder mehrere der folgenden Vorteile beobachtet oder erzielt werden:
- Das Verfahren ist lebensmitteltechnologisch und -rechtlich ohne Bedenken einsetzbar
- Das Verfahren kann mehrfach wiederholt werden, um so die Ausbeute zu steigern
- Das Verfahren erfordert keine ungewöhnlichen Verfahrensschritte oder Prozeduren, so dass es bei herkömmlichen Anlagen problemlos einsetzbar ist
- Das Verfahren liefert ein Produkt mit komplexem Aromenprofil, welches als besonders natürlich beschrieben wird.
- Das Verfahren beruht auf den Einsatz nachwachsender Rohstoffe ohne die Verwendung stochiometrischer Reagenzien.

Die einzelnen Schritte des Verfahrens werden im Folgenden erläutert, wobei einzelne Schritte beliebig miteinander kombinierbar sind und einzelne Unterschritte oder Aspekte isolierbar bevorzugte Ausführungsformen der Erfindung darstellen

### Schritt a) Luftoxidation

Unter dem Term " Arachidonsäure oder Arachidonsäureester enthaltenden Edukts" wird zum einen reine Arachidonsäure (=Z,Z,Z,Z-Eicosa-5,8,11,14-tetraensäure) oder deren Ester, bevorzugt Methyl, Ethyl oder Hydroxyalkylester verstanden, bevorzugte Hydroxyalkylester sind Dihydroxypropylarachidonsäureester.

Dieser Term ist aber nicht darauf beschränkt, es können auch Edukte verwendet werden, die freie Arachidonsäure oder Arachidonsäureester enthalten. Besonders bevorzugt ist dabei die Verwendung eines Öls, welches als wesentlichen Bestandteil der enthaltenen glyceridisch gebundenen Fettsäuren Arachidonsäure ausweist. Bevorzugt werden dabei Öle, die ≥ 20 - ≤ 80 Gew-% gebundener Arachidonsäure noch bevorzugt ≥ 35 - ≤ 45 Gew-% Arachidonsäure enthalten.

Gemäß einer bevorzugten Ausführungsform der vorliegenden Erfindung erfolgt die Luftoxidation durch Begasen mit Luft. Die bevorzugte Luftmenge ist ≥ 10 - ≤ 1000 l/h pro kg Edukt. Dies hat sich als vorteilhaft herausgestellt. Bei geringeren Luftmengen ist oftmals die Ausbeute zu gering, höhere Luftmengen führen zur Bildung von Nebenprodukten oder Zersetzung der entstehenden gewünschten Produkte.

Besonders bevorzugt ist eine Luftmenge von ≥ 30 - ≤ 500 l/h pro kg, am meisten bevorzugt ist ≥40 - ≤100 l/h pro kg Edukt.

Schritt a wird bevorzugt für einen Zeitraum von ≥ 1 - ≤ 20 h, bevorzugt ≥ 2 - ≤ 7h durchgeführt. Längere Reaktionsdauern haben sich oftmals als nachteilig herausgestellt, da so unerwünschte Nebenprodukte in zu großen Mengen entstehen.

Schritt a) wird besonders bevorzugt bei einer Temperatur von ≥ 100 °C und ≤ 200°C durchgeführt.

Dabei sind zwei Temperaturbereiche in Kombinationen mit unterschiedlichen Reaktionsdauern besonders bevorzugt:
Gemäß einer bevorzugten Ausführungsform wird Schritt a) bei einer Temperatur von ≥ 110 °C und ≤ 130°C für ≥ 5 - ≤ 7h durchgeführt.

Alternativ wird gemäß einer bevorzugten Ausführungsform Schritt a) bei einer Temperatur von ≥ 160 °C und ≤ 180°C für ≥ 1 - ≤ 3h durchgeführt.

Schritt a) kann mit reinem Edukt durchgeführt werden. Alternativ wird Schritt a) in Gegenwart eines Lösungsmittels durchgeführt. Bevorzugt sind Triacetin oder Triethylcitrat. Besonders bevorzugt ist Triacetin, bevorzugt in einer Menge von ≥ 2 - ≤ 20 Gew%, bezogen auf das Edukt. Am meisten bevorzugt ist die Zugabe von ≥ 5 - ≤ 15 Gew-% Triacetin.

Gemäß einer weiteren bevorzugten Ausführungsform der Erfindung wird bei der Verwendung eines Öls mit einem Anteil an glyceridisch gebundener Arachidonsäure oder eines anderen Esters der Arachidonsäure vor Schritt a) zusätzlich zu der im Öl gebunden vorliegenden Arachidonsäure noch eine freie ungesättigte Fettsäure, bevorzugt in einem Anteil von ≥5 - ≤10 Gew-% bezogen auf das Edukt zugesetzt. Besonders bevorzugt sind Linolsäure, Linolensäure und Arachidonsäure. Am meisten bevorzugt ist Linolensäure.

### Schritt b)

Schritt b) erfolgt durch Erhitzen auf Temperaturen von ≥ 100 °C und ≤ 200°C. Reaktionsdauern sind ≥ 0,5 - ≤ 5h

Dabei sind zwei Temperaturbereiche in Kombinationen mit unterschiedlichen Reaktionsdauern besonders bevorzugt:
Gemäß einer bevorzugten Ausführungsform wird Schritt b) bei einer Temperatur von ≥ 120 °C und ≤ 140°C für ≥ 1,5 - ≤ 2,5h durchgeführt.

Alternativ wird gemäß einer bevorzugten Ausführungsform Schritt b) bei einer Temperatur von ≥ 160 °C und ≤ 180°C für ≥ 0,5 - ≤ 1,5h durchgeführt.

### Schritt c)

Schritt c) erfolgt bevorzugt mittels fraktionierter Destillation. Dabei kann ggf. gemäß einer bevorzugten Ausführungsform zuvor noch Triacetin bevorzugt in einer Menge von ≥ 2 - ≤ 60 Gew-% besonders bevorzugt ≥ 5 - ≤ 40 Gew-% besonders bevorzugt ≥ 10 - ≤ 20 Gew-%, jeweils bezogen auf die Ausgangssubstanz, zugesetzt werden.

Bevorzugt werden dabei zunächst leicht flüchtige Verbindungen abgetrennt. Dies hat sich oftmals als günstig herausgestellt, da so bei vielen Anwendungen auf einfache Weise unerwünschte grüne Nebennoten der Aromamischung, welche durch vorhandene Hexanal oder 2E-Octenal entstehen, vermieden werden können

Gemäß einer bevorzugten Ausführungsform umfasst das Verfahren noch einen Schritt d)
d) Wiederholen, insbesondere mehrmaliges Wiederholen der Schritte a) bis c) in dieser Reihenfolge.

Bevorzugt wird die Sequenz aus a) bis c) insgesamt drei- bis fünfmal, bevorzugt viermal wiederholt.

Die vorliegende Erfindung bezieht sich außerdem auf eine Aromamischung, herstellbar gemäß dem obig beschriebenen Verfahren, sowie ein Nahrungs- und/oder Nahrungsergänzungsmittel umfassend eine derartige Aromamischung.

Bevorzugt umfasst die Aromamischung eine der Verbindungen 2E,4Z,7Z und 2E,4E,7Z Tridecatrienal.

Die vorliegende Erfindung bezieht sich außerdem auf konfektionierte Produkte/Zubereitungen umfassend eine Aromamischung, hergestellt oder herstellbar gemäß dem vorliegenden Verfahren.

Diese können dabei Nahrungsmittel und/oder Nahrungsergänzungsmittel sein.

Somit bezieht sich die vorliegende Erfindung außerdem ein Nahrungs- und/oder Nahrungsergänzungsmittel umfassend eine erfindungsgemäße Aromamischung.

Bevorzugt ist der Anteil der Aromamischung, bezogen auf das Nahrungs- und/oder Nahrungsergänzungsmittel dabei ≥ 0,0001 Gew.-% (1 ppm) bis ≤ 0,5 Gew.-% (5000 ppm), bevorzugt ≥ 0,0001 Gew.-% (1 ppm) bis ≤ 0,1 Gew.-% (1000 ppm), besonders bevorzugt ≥ 0,001 Gew.-% (10 ppm) bis ≤ 0,05 Gew.-% (500 ppm).

Gemäß einer bevorzugten Ausführungsform beinhalten die Nahrungs- und/oder Nahrungsergänzungsmittel noch flüchtige Aromastoffe.

Bevorzugt beinhalten die Nahrungs- und/oder Nahrungsergänzungsmittel einen oder mehrere Inhaltsstoffe ausgewählt aus der Gruppe enthaltend flüchtige organische Säuren, Alkohole, Thiole und Disulfide, heterocyclische Verbindungen insbesondere Pyridine, Pyrroline, Thiazole und Thiazoline, ferner Aldehyde, Ketone, Ester/Lactone oder Mischungen daraus.

Besonders bevorzugte Inhaltsstoffe - wobei beliebige Inhaltsstoffe miteinander auf beliebige Weise kombiniert werden können - sind dabei:
organische Säuren:
   Essigsäure, Buttersäure, 2- bzw. 3-Methylbuttersäure, Caprinsäure, Capronsäure, Phenylessigsäure;
Alkohole:
   Ethanol, Propylenglykol, 1,3-Octenol, cis-3-Hexenol, Linalool, Benzylalkohol, p-Kresol, 2,6-Dimethylthiophenol, Guajacol, Eugenol,
Disulfide /Thiole:
   Dimethylsulfid, Difurfuryldisulfid, Methylthiopropanal, 2-Methyl-3-methyldithiofuran und Bis(2-methyl-3-furyl)disulfid, Methylfuranthiol, 2-(4-methyl-1,3-thiazol-5-yl)ethanol (Sulfurol), Methyltetrahydrofuranthiol, 3-Methyl-2-buten-1-thiol, 3-Thio-2-methylpentanol, 2-Furfurylthiol, Thiophenol, 2-Methylthiophenol und 2-Mercaptobutanon;
Pyridine:
   2-Acetylpyridin; Pyrazine, weiter bevorzugt Methylpyrazin, 2,5-Methylethylpyrazin, 2,3,5-Trimethylpyrazin, Acetylpyrazin, 2,3-Diethyl-5- methylpyrazin, 2-Ethyl-3,5-dimethylpyrazin und 2-lsopropyl-3-methoxypyrazin;
Thiazole/Thiazoline
   2-Acetylthiazol, 2-Acetyl-2-thiazolin;
Pyrroline:
   2-Propionyl-1-pyrrolin und 2-Acetyl-1-pyrrolin;
   sonstige heterocyclische Verbindungen:
      Indol und Skatol.
Aldehyde:
   Acetaldehyd, trans-4,5-Epoxy-(2E)-decenal, cis-4,5-Epoxy-(2E)-decenal, (E,E)-2,4-Undecadienal, (E,E)-2,4-Decadienal, (E,Z)-2,4-Decadienal, (E,E,Z)-2,4,6-Nonatrienal, (E,E)-2,4-Nonadienal, (E)-2-Undecenal, (Z)-2-Decenal, (E)-2-Decenal, (E)-2-Nonenal, (Z)-2-Nonenal, (E,Z)-2,6-Nonadienal, (E,E)-2,4-Nonadienal, 3-Methylthiopropanal (Methional), Vanillin und Phenylacetaldehyd;
Ketone:
   3,4-Dimethylcyclopentan-1,2-dion, 3-Hydroxy-4,5-dimethylfüran-2(5H)-on (Sotolon), 2-Aminoacetophenon, 3-Hydroxy-4,5-dimethyl-2(5H)-furanon, 2,5-Dimethyl-4-hydroxy-3-[2H]-furanon (Furaneol©), Tetrahydrothiophen-3-on und 3-Thiobutan-2-on;
Ester und Lactone:
   Methyl- butanoat, Ethyl-3-methylbutanoat, Propyl-2-methylbutanoat, (Z)-6-Dodecen-y-lacton, 4-Hydroxy-2-nonensäurelacton, δ-Undecalacton, γ-Nonalacton und γ-Octalacton.

Die Nahrungs- oder Nahrungsergänzungsmittel können gemäß einer bevorzugten Ausführungsform noch weitere Aromastoffe umfassen.

Der oder die Aromastoffe können dabei in Form von Reaktionsaromen (Maillard-Produkte), Extrakten bzw. ätherischen Ölen von Pflanzen oder Pflanzenteilen bzw. Fraktionen davon, Raucharomen oder andere aromagebende Zubereitungen (z.B. Protein[teil]hydrolysaten, Grillaromen, Pflanzenextrakten, Gewürzen, Gewürzzubereitungen, Gemüsesorten und/oder Gemüsezubereitungen eingesetzt werden.

Insbesondere sind hierfür Aromastoffe oder deren Bestandteile geeignet, die nicht in den Gemisch umfasst sind und einen röstigen, fleischigen (insbesondere Huhn, Fisch, Meerestiere, Rind, Schwein, Lamm, Schaf, Ziege), gemüsigen (insbesondere Tomate, Zwiebel, Knoblauch, Sellerie, Lauch, Pilze, Auberginen, Seetang), einen würzigen (insbesondere schwarzer und weißer Pfeffer, Chili, Paprika, Kardamom, Muskat, Piment, Senf und Senf-Produkte), gebratenen, hefigen, gesottenen, fettigen, salzigen und/oder scharfen Aromaeindruck verursachen und somit den würzigen Eindruck verstärken können.

Soweit die vorliegende Erfindung Zubereitungen betrifft, sind diese vorzugsweise eine Zubereitung, die der Ernährung und/oder dem Genuss dient, sowie Halbfertigware für eine Zubereitung, die der Ernährung und/oder dem Genuss dient oder Tiernahrung.

Der Ernährung oder dem Genuss dienende Zubereitungen im Sinne der Erfindung sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kakao, Kaffee, Grüntee, Schwarztee, mit (Grün-, Schwarz-)Tee-Extrakten angereicherte (Grün-, Schwarz-)Tee-Getränke, Roibos-Tee, andere Kräutertee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Pudding, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, isoliertes oder enzymatisch behandeltes Sojaprotein enthaltende Getränke, Sojamehl enthaltende Getränke, Sojalecithinhaltige Zubereitungen, fermentierte Produkte wie Tofu oder Tempe oder daraus gefertigte Produkte und Mischungen mit Fruchtzubereitungen und fakultativ Aromen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, eingekochte Gemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings, jeweils Vollfett- oder fettreduziert), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden, Süßstoffzubereitungen, -tabletten oder -sachets, sonstige Zubereitung zum Süßen oder Weißen von Getränken oder anderen Nahrungsmitteln. Die Zubereitungen im Sinne der Erfindung können auch als Halbfertigware zur Herstellung weiterer der Ernährung oder dem Genuss dienenden Zubereitungen dienen.

Als weitere Bestandteile für erfindungsgemäße Zubereitungen können übliche Grund-, Hilfs- und Zusatzstoffe für Nahrungs- oder Genussmittel verwendet werden. Einige dieser Substanzen besitzen einen unangenehmen Geschmack im Sinne der eingangs gegebenen Definition.

Weitere übliche Wirk-, Grund-, Hilfs- und Zusatzstoffe für der Ernährung oder dem Genuss dienende Zubereitungen können in Mengen von 5 bis 99.999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99.999999 Gew.-%, vorzugsweise 5 bis 80 Gew.-%, bezogen auf das Gesamtgewicht der Zubereitung, aufweisen.
Beispiele üblicher Grund-, Hilfs- und Zusatzstoffe für erfindungsgemäße Zubereitungen sind Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose), Zuckeralkohole (z.B. Sorbit), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. Taurin), Peptide, native oder prozessierte Proteine (z.B. Gelatine), Enzyme (z.B. Peptidasen), Nukleinsäuren, Nucleotide, andere als die erfindungsgemäß verwendeten Geschmackskorrigenzien für unangenehme Geschmackseindrücke (z. B. Hesperetin, Phloretin oder andere gemäß US 2008/0227867 zu verwendende Hydroxychalkonderivate sowie gegebenenfalls die dort beschriebenen Lactone), Geschmackskorrigenzien für weitere, in der Regel nicht unan-genehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure, Milchsäure), zusätzliche Bitterstoffe (z.B. Chinin, Coffein, Limonin, Amarogentin, Humolone, Lupolone, Catechine, Tannine), Süßstoffe (z.B. Saccharin, Cyclamat, Aspartam, Neotam, Stevioside, Rebaudioside, Acesulfam K, Neohesperidindihydrochalkon, Thaumatin, Superaspartam), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, synthetische, natürliche oder naturidentische Aromastoffe oder Riechstoffe sowie Geruchskorrigenzien.

Die vorgenannten sowie die beanspruchten und in den Ausführungsbeispielen beschriebenen erfindungsgemäß zu verwendenden Bauteile unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können.

Weitere Einzelheiten, Merkmale und Vorteile des Gegenstandes der Erfindung ergeben sich aus den Unteransprüchen sowie aus der nachfolgenden Beschreibung der zugehörigen Beispiele, die rein illustrativ und nicht als beschränkend zu verstehen sind:

### Beispiel 1

100 g von Arachidonsäure-haltigen Öl (40 % Arachidonsäure) wird mit 10 g Triacetin verdünnt und bei 120 °C mit Luft (40 L/h) 6 h lang begast. Anschließend wird das Reaktionsgemisch 2 h bei 130 °C erhitzt. Das Rohrprodukt wird im Vakuum destilliert. Zuerst werden die leichtflüchtigen Verbindungen abgetrennt (Bedingungen: 0.1 mbar, 100 °C, 30 min). Das gewünschte Produkt wird anschließend mit Triacetin abdestilliert. (Bedingungen: 0.1 mbar, 170 °C, 1 h (F2)).

Nach destillativer Aufreinigung enthält die erhaltene Lösung (5.7 g) 50 mg von 2E,4Z,7Z und 2E,4E,7Z Tridecatrienal im Verhältnis von 1:1.

### Beispiel 2:

Der Prozess lt. Besipiel 1. wird insgesamt viermal wiederholt. Man erhält 23.3 g Lösung enthaltend 120 mg von 2E,4Z,7Z und 2E,4E,7Z Tridecatrienal im Verhältnis von 1:1.

Diese Mischung wird anschießend noch mit Triacetin verdünnt, sodass eine Mischung entsteht, die insgesamt 0.2 wt% der entsprechenden 2E,4Z,7Z und 2E,4E,7Z Tridecatrienale enthält.

### Beispiel 3:

100 g von Arachidonsäure-haltigen Öl (40 % Arachidonsäure) wird bei 170 °C mit Luft (40 L/h) 2 h lang begast. Anschließend wird das Reaktionsgemisch 1 h bei 170 °C erhitzt. Das Rohrprodukt wird mit 10 g Triacetin verdünnt und im Vakuum destilliert. Zuerst werden die leichtflüchtigen Verbindungen abgetrennt (Bedingungen: 0.1 mbar, 100 °C, 30 min). Das gewünschte Produkt wird anschließend mit Triacetin abdestilliert (Bedingungen: 0.1 mbar, 170 °C, 1 h (F2)).

Nach destillativer Aufreinigung enthält die erhaltene Lösung (7.7 g) 80 mg von 2E,4Z,7Z und 2E,4E,7Z Tridecatrienal im Verhältnis von 1:4.

### Beispiel 4:

Der Prozess aus Beispiel 3 wird insgesamt viermal wiederholt. Man erhält 29.7 g Lösung enthaltend 230 mg von 2E,4Z,7Z und 2E,4E,7Z Tridecatrienal im Verhältnis von 1:4.

Diese Mischung wird anschießend noch mit Triacetin verdünnt, sodass eine Mischung entsteht, die insgesamt 0.2 wt% der entsprechenden 2E,4Z,7Z und 2E,4E,7Z Tridecatrienale enthält.

### Einsatz der Aromamischung:

Im Folgenden werden Verwendungen einer Aromamischung gemäß des erfindungsgemäßen Verfahrens beschrieben. Diese werden - zur Vermeidung von Verwechslungen - als "Kompositionen" bezeichnet.

### Beispiel 1: Herstellung einer Hühnchen - Aromakomposition enthaltend die erfindungsgemäße Aromamischung:

Es werden vermischt:

| Bestandteil | [G] |
|---|---|
| Acetylmethylcarbinol | 0.2 |
| Buttersäure | 2 |
| Capronsäure | 1 |
| Caprylsäure | 1 |
| Decadienal trans, trans- 2,4 | 0.8 |
| 2E-Decenal | 0.2 |
| Dimethyloxyfuranone / Sotolon 1 % Lösung in Alkohol | 0.4 |
| Furaneol 10 % Lösung in Triacetin | 20 |
| Indole 1 % Lösung in Alkohol | 0.4 |
| 2,3-Methylfuranthiol | 0.8 |
| Methyltetrahydrofuranthiol | 0.3 |
| Methylthiopropanal-3 | 0.15 |
| 3,2-Thiobutanon | 1 |
| Reaktionsprodukt aus Beispiel 4 | 15 |
| Pflanzenöltrigyceride | 956.75 |
| Summe | 1000 |

Diese Aromakomposition wird in nachfolgend beschriebenen Anwendungsbeispielen eingesetzt.

### Beispiel 2: Herstellung einer Rindermarkknochen-Aromakomposition

Es werden vermischt:

| **Bestandteil** | **[G]** |
|---|---|
| Acetylmethylcarbinol | 2 |
| Alkohol C6 | 0.5 |
| *p*-Kresol 1 % Lösung in PG | 5 |
| Decadienal trans, trans- 2,4 5% in Triacetine | 50 |
| *delta*-Decalacton | 1.4 |
| *delta-*Dodecalacton | 3.5 |
| Furaneol 10 % Lösung in Triethylcitrat | 30 |
| Furfurylmercaptan 1 % in Triethylcitrat | 3 |
| Indole 1 % in Alkohol | 5 |
| 2,3-Methylfuranthiol 5 % A 1 % in Triacetin | 60 |
| Methylthiopropanal-3 1 % in Triethylcitrat | 15 |
| Myristinsäure | 4 |
| 2,3-Pentandion | 3 |
| 3,2-Thiobutanon 10 % in Pflanzenöltriglyceriden | 12 |
| 2,3,5-Trimethylpyrazin | 1 |
| Reaktionsprodukt aus Beispiel 2 | 4 |
| Pflanzenöltriglyceride | 800.6 |
| Summe | 1000 |

Diese Aromakomposition wird in nachfolgend beschriebenen Anwendungsbeispielen eingesetzt.

### Anwendungsbeispiel 3: Eierpudding Aromakomposition

Es werden vermischt:

| Bestandteil | [G] |
|---|---|
| Acetylpyrazin-2 | 3 |
| Buttersäure | 1 |
| Caprinsäure | 200 |
| Decadienal trans, trans- 2,4 0.01% in Propylenglycol | 0.05 |
| *delta*-Decalacton | 50 |
| Diacetyl | 0.1 |
| *delta-*Dodecalacton | 100 |
| Furaneol 15 % Lösung in Propylenglycol | 0.1 |
| Indole FF 1 % Propylenglycol | 0.75 |
| 2,3-Methylfuranthiol 0.005 % in Triacetin | 40 |
| 1,2-Propylenglycol | 535 |
| Reaktionsprodukt aus Beispiel 4 | 50 |
| Vanillin | 20 |
| Summe | |

Diese Aromakomposition wird in nachfolgend beschriebenen Anwendungsbeispielen eingesetzt.

### Anwendungsbeispiel 4: Sprühgetrocknete Aromakompositionen

Es werden ausgehend von den zuvor beschriebenen Anwendungsbeispielen 1 bis 3 vier Aromakompositionen 4A bis 4C hergestellt, die nachfolgend weiter eingesetzt werden:

| **Inhaltsstoff** | **A** | **B** | **C** |
|---|---|---|---|
| Capsul | 200 g | 200 g | 200 g |
| Maltodextrin | 600 g | 600 g | 600 g |
| Aromakomposition Zusammensetzung Anwendungsbeispiel 1 | 200 g | - | - |
| Aromakomposition Zusammensetzung Anwendungsbeispiel 2 | - | 200 g | - |
| Aromakomposition Zusammensetzung Anwendungsbeispiel 3 | - | - | 200 g |
| Wasser | 1000 g | 1000 g | 1000 g |

Die Bestandteile werden in demineralisiertem Wasser gelöst und anschließend sprühgetrocknet. Diese werden in den nachfolgenden Anwendungsbeispielen verwendet.

### Anwendungsbeispiel 5: Instantsuppe, Typ Hühnersuppe mit Nudeln

A = Vergleichszubereitung
B = erfindungsgemäße Zubereitungen

| **Inhaltsstoff** | **A** | **B** |
|---|---|---|
| Stärke | 16 g | 16 g |
| Kochsalz | 7 g | 7 g |
| Saccharose, raffiniert | 3.2 g | 3.2 g |
| Natriumglutamat | 3.2 g | 3.2 g |
| Natriuminosinat / Natriumguanylat im Verhältnis 1:1 | 0.8 g | 0.8 g |
| Säurehydrolysiertes Pflanzenprotein | 8.0 g | 8.0 g |
| Fettpulver | 2.0 g | 2.0 g |
| Gemüsefett, sprühgetrocknet | 1.0 g | 1.0 g |
| Gefriergetrocknetes Hühnerfleisch, in Stückchen | 2.15 g | 2.15 g |
| Suppen-Nudeln | 32.0 g | 32.0 g |
| Maltodextrin | 12.0 g | 7.6 g |
| Chinesisches Gemüse, gefriergetrocknet | 4.6 g | 4.6 g |
| Huhnaroma | 8.0 g | 6.9 g |
| Lebensmittelfarbstoff Riboflavin | 0.05 g | 0.05 g |
| Sprühgetrocknete Aromakompositionen aus Anwendungsbeispiel 4A | - | 5.5 g |

4,6 g der jeweiligen Pulvermischung werden 10 Minuten in je 100 ml Wasser gekocht, um eine verzehrfertige Suppe zu erhalten. Die erfindungsgemäße Zubereitung B wird in Bezug auf erwünschte fettige Aromen erinnernd an Hähnchenhaut als stärker und lang anhaltender bewertet, was ein deutlich authentischeres Profil ergibt.

### Anwendungsbeispiel 6: Instantsuppe, Typ Rindfleischsuppe mit Nudeln

A = Vergleichszubereitung
B = erfindungsgemäße Zubereitungen

| **Inhaltsstoff** | **A** | **B** |
|---|---|---|
| Stärke | 16 g | 16 g |
| Kochsalz | 7 g | 7 g |
| Saccharose, raffiniert | 3.2 g | 3.2 g |
| Natriumglutamat | 3.2 g | 3.2 g |
| Natriuminosinat / Natriumguanylat im Verhältnis 1:1 | 0.8 g | 0.8 g |
| Säurehydrolysiertes Pflanzenprotein | 8.0 g | 8.0 g |
| Fettpulver | 2.0 g | 2.0 g |
| Gemüsefett, sprühgetrocknet | 1.0 g | 1.0 g |
| Gefriergetrocknetes Rinderfleisch, in Stückchen | 2.15 g | 2.15 g |
| Suppen-Nudeln | 32.0 g | 32.0 g |
| Maltodextrin | 12.0 g | 11.1 g |
| Gemüse, gefriergetrocknet | 4.6 g | 4.6 g |
| Fleischaroma | 8.0 g | 7.8 g |
| Lebensmittelfarbstoff Riboflavin | 0.05 g | 0.05 g |
| Sprühgetrocknete Aromakompositionen Anwendungsbeispiel 4B | - | 1.1 g |

4,6 g der jeweiligen Pulvermischung wird 10 Minuten in je 100 ml Wasser gekocht, um eine verzehrfertige Suppe zu erhalten. Durch die Verwendung der erfindungsgemäßen Aromakomposition mit Tridecatrienal wird ein fettiger, vollmundiger Geschmack mit authentischem Profil erreicht, welches bei der Verkostung durch ein Panel ausgebildeter Testpersonen gegenüber Zubereitung A bevorzugt wurde.

### Anwendungsbeispiel 7: Bouillon

A = Vergleichszubereitung
B, C = erfindungsgemäße Zubereitungen

| **Inhaltsstoff** | **A** | **B** | **C** |
|---|---|---|---|
| Fettpulver | 8.77 g | 8.77 g | 8.77 g |
| Natriumglutamat | 8.77 g | 8.77 g | 8.77 g |
| Hefeextrakt Pulver | 12.28 g | 12.28 g | 12.28 g |
| Kochsalz | 29.83 g | 29.83 g | 29.83 g |
| Maltodextrin | 37.28 g | 20.78 g | 33.78 g |
| Natürlicher Gemüseextrakt | 3.07 g | 3.07 g | 3.07 g |
| Sprühgetrocknete Aromakompositionen Anwendungsbeispiel 4A | - | 16.5 g | - |
| Sprühgetrocknete Aromakompositionen Anwendungsbeispiel 4B | - | - | 3.5 g |

15 g der jeweiligen Pulvermischung wird mit je 1000 ml heißem Wasser aufgegossen. Bei der Verkostung durch ein Panel ausgebildeter Testpersonen wird die erfindungsgemäßen Zubereitungen B und C hinsichtlich ihres Aroma und Geschmack als deutlich reicher, ausgewogener, kräftiger und langanhaltender als die Vergleichszubereitung A bewertet.

### Anwendungsbeispiel 8: Vanille Kochpudding

A = Vergleichszubereitung
B = erfindungsgemäße Zubereitungen

| **Inhaltsstoff** | **A** | **B** |
|---|---|---|
| Sucrose | 7.8 g | 7.8 g |
| Stärke | 3.0 g | 3.0 g |
| Magermilch-pulver | 1.5 g | 1.5 g |
| Aubygel MR50 | 0.5 g | 0.5 g |
| Vanilleschoten-Extrakt, sprühgetrocknet, Symrise | 0.1 g | 0.1 g |
| Sprühgetrocknete Aromakompositionen Anwendungsbeispiel 4C | - | 0.25 g |
| Milch 1,5 % Fettanteil | ad 100 g | |

Die festen Stoffe werden vorgelegt und mit der Milch aufgerührt. Die Mischung wird auf 95 °C für 2 min unter gutem Rühren aufgewärmt, abgefüllt und auf 5 - 8 °C abgekühlt. Durch die Verwendung der erfindungsgemäßen Aromakomposition mit Tridecatrienal wird deutlich fettiger, authentischer Geschmack mit ausgeprägter Ei-note erreicht.

### Anwendungsbeispiel 9: Weiße Soße

A = Vergleichszubereitung
B, C = Vergleichszubereitung

| **Bestandteil** | **A** | **B** | **C** |
|---|---|---|---|
| Maltodextrin | 26.00 g | 23.25 g | 25.40 g |
| Kochsalz | 7.50 g | 7.50 g | 7.50 g |
| Natriumglutamat | 2.00 g | 2.00 g | 2.00 g |
| Pflanzenfett | 5.00 g | 5.00 g | 5.00 g |
| Pfeffer, weiß | 0.02 g | 0.02 g | 0.02 g |
| Zwiebelpulver | 1.48 g | 1.48 g | 1.48 g |
| vorverkleisterte Maisstärke | 30.00 g | 30.00 g | 30.00 g |
| Fettpulver | 28.00 g | 28.00 g | 28.00 g |
| Sprühgetrocknete Zusammensetzung Anwendungsbeispiel 4A | - | 2.75 g | - |
| Sprühgetrocknete Zusammensetzung Anwendungsbeispiel 4B | - | - | 0.6 g |

90 g der Soßenmischung wird mit 1000 ml heißem Wasser aufgegossen und mit dem Schneebesen kräftig verrührt. Bei der Verkostung durch ein Panel ausgebildeter Testpersonen werden die erfindungsgemäßen Zubereitungen B und C hinsichtlich ihres Aroma und Geschmack als deutlich reicher, ausgewogener, kräftiger und langanhaltender als die Vergleichszubereitung A bewertet.

### Anwendungsbeispiel 10: Braune Soße

A = Vergleichszubereitung
B = Vergleichszubereitung

| **Bestandteil** | **A** | **B** | **C** |
|---|---|---|---|
| Stärke | 40.00 g | 40.00 g | 40.00 g |
| Maltodextrin | 33.10 g | 30.35 g | 32.50 g |
| Kochsalz | 6.00 g | 6.00 g | 6.00 g |
| Zuckerkulör, sprühgetrocknet | 5.00 g | 5.00 g | 5.00 g |
| Hefeextraktpulver | 3.00 g | 3.00 g | 3.00 g |
| Natriumglutamat | 2.00 g | 2.00 g | 2.00 g |
| Zucker | 0.50 g | 0.50 g | 0.50 g |
| Fettpulver | 5.00 g | 5.00 g | 5.00 g |
| Tomatenpulver | 3.00 g | 3.00 g | 3.00 g |
| Natürlicher Gemüseextrakt | 1.00 g | 1.00 g | 1.00 g |
| Zwiebelextrakt | 0.30 g | 0.30 g | 0.30 g |
| Pfefferextrakt | 0.10 g | 0.10 g | 0.10 g |
| Trockenaroma | 1.00 g | 1.00 g | 1.00 g |
| Sprühgetrocknete Zusammensetzung Anwendungsbeispiel 4A | - | 2.75 g | - |
| Sprühgetrocknete Zusammensetzung Anwendungsbeispiel 4B | | - | 0.6 g |

90 g der Soßenmischung werden mit 1000 ml heißem Wasser aufgegossen und mit dem Schneebesen kräftig verrührt. Bei der Verkostung durch ein Panel ausgebildeter Testpersonen werden die erfindungsgemäßen Zubereitungen B und C hinsichtlich ihres Aroma und Geschmack als deutlich reicher, ausgewogener, kräftiger und langanhaltender als die Vergleichszubereitung A bewertet.

### Anwendungsbeispiel 11: Würzmischung für Kartoffelchips

A = Vergleichszubereitung
B = erfindungsgemäße Zubereitungen

| **Bestandteil** | **A** | **B** |
|---|---|---|
| Natriumglutamat | 3.50 g | 3.50 g |
| Käsepulver | 10.00 g | 10.00 g |
| Knoblauchpulver | 2.00 g | 2.00 g |
| Molkenpulver | 38.86 g | 36.86 g |
| Würzextraktöl | 0.20 g | 0.20 g |
| Paprikapulver | 9.80 g | 9.80 g |
| Kochsalz | 21.00 g | 19.00 g |
| Tomatenpulver | 9.00 g | 9.00 g |
| Trockenaroma | 2.50 g | 2.50 g |
| Siliciumdioxid | 0.02 g | 0.02 g |
| Pflanzenöl | 0.02 g | 0.02 g |
| Zwiebelpulver | 3.00 g | 3.00 g |
| Sahne Aromakonzentrat | 0.03 g | 0.03 g |
| Käse Aroma | 0.03 g | 0.03 g |
| Tomaten Aromakonzentrat | 0.04 g | 0.04 g |
| Sprühgetrocknete Zusammensetzung Anwendungsbeispiel 4A | - | 4.00 g |

6 g der Würzmischung werden auf 94 g Kartoffelchips aufgezogen.

### Anwendungsbeispiel 12: Rindfleischwürzmischung für (Fertig)-Nudeln

| **Inhaltsstoff** | **Gew.-%** |
|---|---|
| Rindsfettaroma | 5.00 |
| Zuckercouleur | 3.00 |
| Zitronensäure (wasserfrei) | 0.40 |
| Schnittlauch (entwässert) | 2.00 |
| Maltodextrin (ex Tapoica) | 10.30 |
| Mononatriumglutamat | 15.00 |
| Zwiebelpulver | 5.00 |
| Ribotide | 0.80 |
| Natriumchlorid | 44.20 |
| Zucker | 2.80 |
| Süßmolkepulver | 6.50 |
| Sprühgetrocknete Zusammensetzung Anwendungsbeispiel 4B | 5.00 |

Alle Inhaltsstoffe werden vermischt, bis sich eine homogene Mischung ergibt.

### Anwendungsbeispiel 13: Hähnchenfleischwürzmischung für (Fertig)-Nudeln

| **Inhaltsstoff** | **Gew.-%** |
|---|---|
| Hähnchenaroma | 5.00 |
| Zuckercouleur | 3.00 |
| Zitronensäure (wasserfrei) | 0.40 |
| Schnittlauch (entwässert) | 2.00 |
| Maltodextrin (ex Tapoica) | 5.30 |
| Mononatriumglutamat | 15.00 |
| Zwiebelpulver | 5.00 |
| Ribotide | 0.80 |
| Natriumchlorid | 39.20 |
| Zucker | 2.80 |
| Süßmolkepulver | 6.50 |
| Sprühgetrocknete Zusammensetzung Anwendungsbeispiel 4A | 15.00 |

Alle Inhaltsstoffe werden vermischt, bis sich eine homogene Mischung ergibt.

Die einzelnen Kombinationen der Bestandteile und der Merkmale von den bereits erwähnten Ausführungen sind exemplarisch; der Austausch und die Substitution dieser Lehren mit anderen Lehren, die in dieser Druckschrift enthalten sind mit den zitierten Druckschriften werden ebenfalls ausdrücklich erwogen. Der Fachmann erkennt, dass Variationen, Modifikationen und andere Ausführungen, die hier beschrieben werden, ebenfalls auftreten können.

Entsprechend ist die obengenannte Beschreibung beispielhaft und nicht als beschränkend anzusehen. Das in den Ansprüchen verwendete Wort "umfassen" schließt nicht andere Bestandteile oder Schritte aus. Der unbestimmte Artikel "ein" schließt nicht die Bedeutung eines Plurals aus. Die bloße Tatsache, dass bestimmte Maße in gegenseitig verschiedenen Ansprüchen rezitiert werden, verdeutlicht nicht, dass eine Kombination von diesen Maßen nicht zum Vorteil benutzt werde kann. Der Umfang der Erfindung ist in den folgenden Ansprüchen defniert.

## Patentansprüche

1. Verfahren zum Herstellen ungesättigter Decanale umfassend die Schritte
a) Luftoxidation eines Arachidonsäure oder Arachidonsäureester enthaltenden Edukts
b) Zersetzung des entstehenden Peroxids
c) Aufkonzentrierung,
wobei Schritt b) durch Erhitzen bei einer Temperatur von ≥ 100°C bis ≤ 200°C durchgeführt wird,
und wobei Schritt b) für ≥ 0,5h - ≤ 5h erfolgt.

2. Verfahren nach Anspruch 1, zusätzlich umfassend den Schritt d) d) Wiederholen der Schritte a) bis c) in dieser Reihenfolge.

3. Verfahren nach einem der Ansprüche 1 oder 2, wobei Schritt a) das Begasen mit Luft bei einer Luftmenge von ≥ 10 - ≤ 1000 l/h pro kg umfasst.

4. Verfahren nach einem der Ansprüche 1 bis 3, wobei Schritt a) bei einer Temperatur von ≥ 100°C und ≤ 200°C durchgeführt wird.

5. Verfahren nach einem der Ansprüche 1 bis 4, wobei Schritt a) für eine Dauer von ≥ 1h bis ≤ 20h durchgeführt wird.

6. Verfahren nach einem der Ansprüche 1 bis 5, wobei Schritt c) eine fraktionierte Destillation enthält.

7. Aromamischung, herstellbar durch ein Verfahren gemäß der Ansprüche 1 bis 6.

8. Nahrungs- oder Nahrungsergänzungsmittel, umfassend eine Aromamischung gemäß Anspruch 7.

## Claims

1. A method for manufacturing unsaturated decanals comprising the steps
(a) air oxidation of an educt containing arachidonic acid or arachidonic acid ester
(b) decomposition of the resulting peroxide
(c) concentration,
wherein step b) is performed by heating at a temperature of ≥ 100°C to ≤ 200°C, and wherein step b) takes place for ≥ 0.5h - ≤ 5h.

2. The method according to claim 1, additionally comprising the step d) d) repeating the steps a) to c) in this order.

3. The method according to any one of claims 1 or 2, wherein step a) comprises gassing with air at an air volume of ≥ 10 - ≤ 1000 l/h per kg.

4. The method according to any one of claims 1 to 3, wherein step a) is performed at a temperature of ≥ 100°C and ≤ 200°C.

5. The method according to any one of claims 1 to 4, wherein step a) is performed for a duration of ≥ 1h to ≤ 20h.

6. The method according to any one of claims 1 to 5, wherein step c) comprises a fractionated distillation.

7. An aroma mixture producible by a method according to claims 1 to 6.

8. A food or food supplement comprising an aroma mixture according to claim 7.

## Revendications

1. Procédé de préparation de composés décanal insaturés, comprenant les étapes
a) oxydation à l'air d'un éduit contenant de l'acide arachidonique ou un ester d'acide arachidonique
b) décomposition du peroxyde formé
c) augmentation de concentration,
dans lequel l'étape b) est mise en œuvre par chauffage à une température comprise entre ≥ 100 °C et ≤ 200 °C,
et dans lequel l'étape b) se fait pour une durée comprise entre ≥ 0,5h et ≤ 5h.

2. Procédé selon la revendication 1, comprenant en outre l'étape d) d) répéter les étapes a) à c) dans cet ordre.

3. Procédé selon l'une quelconque des revendications 1 ou 2, dans lequel l'étape a) comprend le gazage avec de l'air à une quantité d'air comprise entre ≥ 10 et ≤ 1000 l/h par kg.

4. Procédé selon l'une quelconque des revendications 1 à 3, dans lequel l'étape a) est mise en œuvre à une température comprise entre ≥ 100 °C et ≤ 200 °C.

5. Procédé selon l'une quelconque des revendications 1 à 4, dans lequel l'étape a) est mise en œuvre pour une durée comprise entre ≥ 1h et ≤ 20h.

6. Procédé selon l'une quelconque des revendications 1 à 5, dans lequel l'étape c) contient une distillation fractionnée.

7. Mélange d'arômes apte à être préparé par un procédé selon les revendications 1 à 6.

8. Aliment ou complément alimentaire comprenant un mélange d'arômes selon la revendication 7.
